# EUROPEAN PATENT APPLICATION

(11) **EP 4 574 053 A1**
(43) Date of publication of application: **25.06.2025**
(21) Application number: 23877419.4
(22) Date of filing: 21.06.2023
(51) Int. Cl.: A61B 8/08, G16H 50/20, G16H 30/40, G06N 3/08

(54) **BLOOD VESSEL DETECTION METHOD, AND COMPUTER PROGRAM PERFORMING SAME**

(30) Priority: 14.10.2022 KR 20220132183
(71) Applicant: Airs Medical Inc., Seoul 08788 (KR)
(72) Inventor: KIM, Youngsik, Seoul 06571 (KR); NAM, Deokgeun, Siheung-si Gyeonggi-do 14991 (KR)
(74) Representative: Isarpatent
(86) International application number: PCT/KR2023/008615
(87) International publication number: WO 2024/080488

(57) **Abstract**

A blood vessel detection method that is performed by a computing device including at least one processor according to one embodiment of the present disclosure includes collecting the medical image of an n-th frame (n is a natural number) from an image acquisition device, and detecting a first blood vessel region from the medical image by using trained first and second models and detecting a second blood vessel region from the medical image by using the first model, and collecting the medical image and detecting the first and second blood vessel regions are repeated for a preset time.

## Description

### Technical Field

The present disclosure relates to a blood vessel detection method and a computer program performing the same, and more particularly, to a blood vessel detection method using neural network models and a computer program performing the same.

### Background Art

Medical images are data that allows users to understand the physical states of various organs in the human body for the diagnosis and treatment of diseases. As medical imaging devices continue to be developed, medical images are becoming more diverse, as in the case of ultrasonic images, X-ray images, computed tomography (CT) images, positron emission tomography (PET) images, or magnetic resonance imaging (MRI) images.

Among these types of medical images, ultrasonic images include gray-scale ultrasonic images and Doppler ultrasonic images. In particular, Doppler ultrasonic images enable not only the imaging of the inside structures of blood vessels but also the measurement of the blood flow inside blood vessels. More specifically, they enable the measurement of the blood flow velocity and blood flow in veins and the measurement of the resistance index and pulsatility index in arteries.

Ultrasonic images have the characteristic of being acquired in real time. Furthermore, acquired images vary depending on changes in the body, such as the beating of blood vessels, so that in the case of technologies targeting ultrasonic images, it is necessary to consistently analyze images that vary in real time. In particular, due to the nature of the medical field, there is a difficulty in achieving a high level of analysis accuracy while satisfying the analysis speed corresponding to image acquisition.

### Disclosure

### Technical Problem

The present disclosure is intended to overcome the above-described problems of the conventional art, and is directed to a blood vessel detection method using a plurality of neural network models to detect blood vessels and a computer program performing the same.

However, the technical problems to be overcome by the present embodiment are not limited to the technical problem described above, and other technical problems may be present.

### Technical Solution

According to one embodiment of the present disclosure for achieving the above-described object, there is disclosed a blood vessel detection method that is performed by a computing device. The blood vessel detection method includes collecting the medical image of an n-th frame (n is a natural number) from an image acquisition device, and detecting a first blood vessel region from the medical image by using trained first and second models and detecting a second blood vessel region from the medical image by using the first model, and collecting the medical image and detecting the first and second blood vessel regions are repeated for a preset time.

Alternatively, detecting the first and second blood vessel regions may include: detecting a first blood vessel candidate region from the medical image by using the first model; setting a box including the first blood vessel candidate region when the first blood vessel candidate region is larger than a reference value; and detecting the first blood vessel region from the box by using the second model.

Alternatively, the blood vessel detection method may further include, after detecting the first and second blood vessel regions, displaying the first and second blood vessel regions, and collecting the medical image to displaying the first and second blood vessel regions are repeated for the preset time.

Alternatively, displaying the first and second blood vessel regions may include: comparing the location of the first blood vessel region with the location of a first blood vessel region of the medical image of a m-th frame (m is a natural number smaller than n), and displaying the first blood vessel region based on comparison results.

Alternatively, displaying the first and second blood vessel regions may include: comparing the location of the second blood vessel region with the location of a second blood vessel region of the medical image of the m-th frame, and displaying the second blood vessel region based on comparison results.

Alternatively, displaying the first and second blood vessel regions may include: calculating at least one of the maximum blood vessel cross-sectional area, the degree of blood vessel expansion, and whether a blood vessel is compressed for each of the first and second blood vessel regions; and displaying at least one of the maximum blood vessel cross-sectional area, the degree of blood vessel expansion, and whether the blood vessel is compressed.

Alternatively, the blood vessel detection method may further include, after the preset time has ended, detecting a final first blood vessel region from a plurality of medical images accumulated during the preset time by using the first model, and detecting a final second blood vessel region from the plurality of accumulated medical images by using a trained third model.

Alternatively, the third model may receive a plurality of medical images extracted from among the plurality of accumulated medical images.

Alternatively, the image acquisition device may collect the medical image based on a condition control algorithm as at least one of the hardware characteristics and software characteristics of the image acquisition device is changed.

Alternatively, the hardware characteristics may include at least one of the location of the image acquisition device, the distance from a patient, a body compression intensity for the patient, a compression direction, and a compression time, and the software characteristics may include at least one of the time taken for acquiring the medical image, and the resolution and size of the medical image.

Alternatively, the first and second blood vessels may be a vein and an artery, respectively, or an artery and a vein, respectively.

According to one embodiment of the present disclosure for achieving the above-described object, there is disclosed a blood vessel detection method that is performed by a computing device. The blood vessel detection method includes: detecting a final first blood vessel region from a plurality of medical images by using a trained first model, and detecting a final second blood vessel region from the plurality of medical images by using a trained second model; and displaying the final first and second blood vessel regions.

Alternatively, the blood vessel detection method may further include, before detecting the final first and second blood vessel regions, detecting first and second blood vessel regions for a preset time, and the plurality of medical images may be acquired for the preset time.

Alternatively, detecting the first and second blood vessel regions may include detecting the first blood vessel region from a medical image by using the first model and a trained third model and detecting the second blood vessel region from the medical image by using the first model.

Alternatively, displaying the final first and second blood vessel regions may include displaying a region in which the final first and second blood vessel regions overlap each other as the final second blood vessel region.

Alternatively, the trained first model may receive any one of the plurality of medical images.

Alternatively, the trained second model may receive a plurality of medical images extracted from among the plurality of medical images.

Alternatively, the plurality of medical images may be collected based on a condition control algorithm as at least one of the hardware characteristics and software characteristics of an image acquisition device is changed.

Alternatively, the first and second blood vessels may be a vein and an artery, respectively, or an artery and a vein, respectively.

According to one embodiment of the present disclosure for achieving the above-described object, there is disclosed a computer program stored in a computer-readable storage medium. When executed on at least one processor, the computer program causes the processor to perform operations. The operations include: collecting a medical image from an image acquisition device; detecting a first blood vessel region from the medical image by using a trained first model and a trained second model, and detecting a second blood vessel region from the medical image by using the first model; displaying the first blood vessel region and the second blood vessel region; repeating collecting the medical image and detecting the first and second blood vessel regions for a preset time; and, after the preset time has ended, detecting a final first blood vessel region from a plurality of medical images accumulated during the preset time by using the first model, and detecting a final second blood vessel region from the plurality of accumulated medical images by using the trained third model.

### Advantageous Effects

According to the above-described technical solution of the present disclosure, the present disclosure takes into consideration the characteristics of blood vessels varying depending on the imaging environment by performing real-time blood vessel detection, so that the accuracy of blood vessel detection can be increased even in a situation in which it is difficult to acquire consistent images.

Furthermore, according to the above-described technical solution of the present disclosure, the present disclosure detects blood vessels using a plurality of neural network models, so that blood vessel detection can be performed with different characteristics taken into consideration depending on the type of blood vessels.

### Description of Drawings

FIG. 1 is a block diagram of a computing device according to one embodiment of the present disclosure;
FIG. 2 is a block diagram of a blood vessel detection apparatus according to one embodiment of the present disclosure;
FIG. 3 is a block diagram of a first model according to one embodiment of the present disclosure;
FIG. 4 is a block diagram of a second model according to one embodiment of the present disclosure;
FIG. 5 is a block diagram of a third model according to one embodiment of the present disclosure;
FIG. 6 is a flowchart showing the operation method of a blood vessel detection apparatus according to one embodiment of the present disclosure;
FIG. 7 is a flowchart showing a real-time blood vessel detection method according to one embodiment of the present disclosure;
FIG. 8 is a flowchart showing in detail a real-time blood vessel detection method according to one embodiment of the present disclosure; and
FIG. 9 is a flowchart showing a final blood vessel detection method according to one embodiment of the present disclosure.

### Mode for Invention

Embodiments of the present disclosure will be described in detail below with reference to the accompanying drawings so that those having ordinary skill in the art of the present disclosure (hereinafter referred to as those skilled in the art) can easily implement the present disclosure. The embodiments presented in the present disclosure are provided to enable those skilled in the art to use or practice the content of the present disclosure. Accordingly, various modifications to embodiments of the present disclosure will be apparent to those skilled in the art. That is, the present disclosure may be implemented in various different forms and is not limited to the following embodiments.

The same or similar reference numerals denote the same or similar components throughout the specification of the present disclosure. Additionally, in order to clearly describe the present disclosure, reference numerals for parts that are not related to the description of the present disclosure may be omitted in the drawings.

The term "or" used herein is intended not to mean an exclusive "or" but to mean an inclusive "or." That is, unless otherwise specified herein or the meaning is not clear from the context, the clause "X uses A or B" should be understood to mean one of the natural inclusive substitutions. For example, unless otherwise specified herein or the meaning is not clear from the context, the clause "X uses A or B" may be interpreted as any one of a case where X uses A, a case where X uses B, and a case where X uses both A and B.

The term "and/or" used herein should be understood to refer to and include all possible combinations of one or more of listed related concepts.

The terms "include" and/or "including" used herein should be understood to mean that specific features and/or components are present. However, the terms "include" and/or "including" should be understood as not excluding the presence or addition of one or more other features, one or more other components, and/or combinations thereof.

Unless otherwise specified herein or unless the context clearly indicates a singular form, the singular form should generally be construed to include "one or more."

The term "N-th (N is a natural number)" used herein may be understood as an expression used to distinguish the components of the present disclosure according to a predetermined criterion such as a functional perspective, a structural perspective, or the convenience of description. For example, in the present disclosure, components performing different functional roles may be distinguished as a first component or a second component. However, components that are substantially the same within the technical spirit of the present disclosure but should be distinguished for the convenience of description may also be distinguished as a first component or a second component.

Meanwhile, the term "module" or "unit" used herein may be understood as a term referring to an independent functional unit processing computing resources, such as a computer-related entity, firmware, software or part thereof, hardware or part thereof, or a combination of software and hardware. In this case, the "module" or "unit" may be a unit composed of a single component, or may be a unit expressed as a combination or set of multiple components. For example, in the narrow sense, the term "module" or "unit" may refer to a hardware component or set of components of a computing device, an application program performing a specific function of software, a procedure implemented through the execution of software, a set of instructions for the execution of a program, or the like. Additionally, in the broad sense, the term "module" or "unit" may refer to a computing device itself constituting part of a system, an application running on the computing device, or the like. However, the above-described concepts are only examples, and the concept of "module" or "unit" may be defined in various manners within a range understandable to those skilled in the art based on the content of the present disclosure.

The term "model" used herein may be understood as a system implemented using mathematical concepts and language to solve a specific problem, a set of software units intended to solve a specific problem, or an abstract model for a process intended to solve a specific problem. For example, a neural network "model" may refer to an overall system implemented as a neural network that is provided with problem-solving capabilities through training. In this case, the neural network may be provided with problem-solving capabilities by optimizing parameters connecting nodes or neurons through training. The neural network "model" may include a single neural network, or a neural network set in which multiple neural networks are combined together.

The term "image" used herein may refer to multidimensional data composed of discrete image elements. In other words, the "image" may be understood as a term referring to a digital representation of an object that can be seen by the human eye. For example, the "image" may refer to multidimensional data composed of elements corresponding to pixels in a two-dimensional image. The "image" may refer to multidimensional data composed of elements corresponding to voxels in a three-dimensional image.

The term "frame" used herein may refer to a discrete data element that constitutes an image or a collection of images. For example, the "frame" may correspond to each image that represents a particular scene in a single image according to a sequential position. Furthermore, the "frame" may correspond to each image that constitutes a part of an image sequence that is a collection of a plurality of images.

The foregoing descriptions of the terms are intended to help to understand the present disclosure. Accordingly, it should be noted that unless the above-described terms are explicitly described as limiting the content of the present disclosure, they are not used in the sense of limiting the technical spirit of the present disclosure.

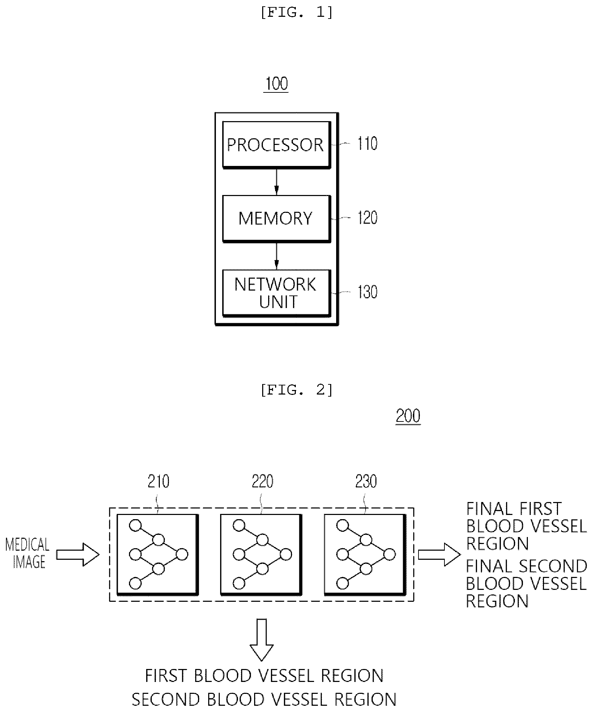

FIG. 1 is a block diagram of a computing device according to one embodiment of the present disclosure.

A computing device 100 according to one embodiment of the present disclosure may be a hardware device or a part of a hardware device that performs the comprehensive processing and computation of data, or may be a software-based computing environment connected to a communication network. For example, the computing device 100 may be a server, which is a main agent that performs intensive data processing functions and shares resources, or may be a client that shares resources through interaction with a server. Furthermore, the computing device 100 may be a cloud system that allows pluralities of servers and clients to comprehensively process data while interacting with each other. Additionally, the computing device 100 may be a medical robot that supports or assists with overall medical procedures performed in a medical field. In this case, the medical robot may include a venipuncture robot that includes a blood collection or intravenous injection (IV) function for diagnosing diseases, transfusions, etc. The above description is only one example related to the type of computing device 100, so that the type of computing device 100 can be configured in various manners within a range understandable to those skilled in the art based on the contents of the present disclosure. The above description is only one example related to the type of computing device 100, so that the type of computing device 100 can be configured in various manners within a range understandable to those skilled in the art based on the contents of the present disclosure.

Referring to FIG. 1, the computing device 100 according to one embodiment of the present disclosure may include a processor 110, memory 120, and a network unit 130. However, FIG. 1 shows only an example, and the computing device 100 may include other components for implementing a computing environment. Alternatively, only some of the components disclosed above may be included in the computing device 100.

The processor 110 according to one embodiment of the present disclosure may be understood as a constituent unit including hardware and/or software for performing computing operation. For example, the processor 110 may read a computer program and perform data processing for machine learning. The processor 110 may process operation processes such as the processing of input data for machine learning, the extraction of features for machine learning, and the computation of errors based on backpropagation. The processor 110 for performing such data processing may include a central processing unit (CPU), a general purpose graphics processing unit (GPGPU), a tensor processing unit (TPU), an application specific integrated circuit (ASIC), or a field programmable gate array (FPGA). Since the types of processor 110 described above are only examples, the type of processor 110 may be configured in various manners within a range understandable to those skilled in the art based on the content of the present disclosure.

The processor 110 may detect blood vessels from medical images by using at least one neural network model. The blood vessels include veins or arteries, and may also include capillaries. The medical images may be images of the upper arm area of the body, but are not limited thereto.

The processor 110 may obtain medical images for a preset time and detect blood vessels for the preset time. In this case, the processor 110 may output and display detection results in real time. Furthermore, the processor 110 may detect blood vessels from a plurality of accumulated medical images after the preset time has ended. In this case, the processor 110 may finally output and display the detection results.

The processor 110 may use the same neural network model for a real-time blood vessel detection operation and a final blood vessel detection operation, or may use different neural network models. For example, the processor 110 may use a fast neural network model for real-time blood vessel detection, and may use a high-accuracy neural network model for final blood vessel detection.

The processor 110 may use a neural network model regardless of the type of blood vessels, or may use different neural network models depending on the type of blood vessels. For example, a neural network model used to detect first blood vessels and a neural network model used to detect second blood vessels may be the same or different. In the present specification, the first blood vessels and the second blood vessels may be arteries and veins, or veins and arteries, respectively. The reason for using different neural network models depending on the type of blood vessels is that the image characteristics also change depending on the characteristics of the blood vessels. More specifically, arteries have the characteristic of not being easily compressed by pressure and thus maintaining their shape, while veins have the characteristic of being swollen by upper arm compression and being compressed when pressure is applied.

The processor 110 may prepare different types of training data to train at least one neural network model. For example, the processor 110 may input training data of a larger size to a neural network model that detects first blood vessel regions and second blood vessel regions than a neural network model that detects only first blood vessel regions. Furthermore, the processor 110 may input training data, obtained by accumulating a plurality of medical images along the time axis, to the neural network model that detects only the second blood vessel regions.

The processor 110 may train the neural network model through supervised learning using learning data as input values. Alternatively, the neural network model may be trained through unsupervised learning that discovers criteria for data recognition by learning the types of data required for data recognition on its own without any guidance. Alternatively, the neural network model may be trained through reinforcement learning that uses feedback on whether the results of data recognition according to learning are correct. The neural network model may include at least one neural network.

The neural network may include network models such as a deep neural network (DNN), a recurrent neural network (RNN), a bidirectional recurrent deep neural network (BRDNN), a multilayer perceptron (MLP), and a convolutional neural network (CNN), but is not limited thereto.

The memory 120 according to one embodiment of the present disclosure may be understood as a constituent unit including hardware and/or software for storing and managing data that is processed in the computing device 100. That is, the memory 120 may store any type of data generated or determined by the processor 110 and any type of data received by the network unit 130. For example, the memory 120 may include at least one type of storage medium of a flash memory type, hard disk type, multimedia card micro type, and card type memory, random access memory (RAM), static random access memory (SRAM), read-only memory (ROM), electrically erasable programmable read-only memory (EEPROM), programmable read-only memory (PROM), magnetic memory, a magnetic disk, and an optical disk. Furthermore, the memory 120 may include a database system that controls and manages data in a predetermined system. Since the types of memory 120 described above are only examples, the type of memory 120 may be configured in various manners within a range understandable to those skilled in the art based on the content of the present disclosure.

The memory 120 may structure, organize and manage data required for the processor 110 to perform operations, combinations of data, and program codes executable by the processor 110. Furthermore, the memory 120 may store program codes that cause the processor 110 to operate to generate training data.

The memory 120 may store collected medical images, may store first blood vessel regions and second blood vessel regions generated as a result of a real-time blood vessel detection operation, and may store final first blood vessel regions and final second blood vessel regions generated as a result of a final blood vessel detection operation.

The network unit 130 according to one embodiment of the present disclosure may be understood as a constituent unit that transmits and receives data through any type of known wired/wireless communication system. For example, the network unit 130 may perform data transmission and reception using a wired/wireless communication system such as a local area network (LAN), a wideband code division multiple access (WCDMA) network, a long term evolution (LTE) network, the wireless broadband Internet (WiBro), a 5th generation mobile communication (5G) network, an ultra-wideband wireless communication network, a ZigBee network, a radio frequency (RF) communication network, a wireless LAN, a wireless fidelity network, a near field communication (NFC) network, or a Bluetooth network. Since the above-described communication systems are only examples, the wired/wireless communication system for the data transmission and reception of the network unit 130 may be applied in various manners other than the above-described examples.

The network unit 130 may receive the data, required for the processor 110 to perform operations, through wired or wireless communication with any system, any client, or the like. Furthermore, the network unit 130 may transmit the data, generated through the operation of the processor 110, through wired or wireless communication with any system, any client, or the like. For example, the network unit 130 may receive multimodal data through communication with a cloud server that performs tasks such as the standardization of medical data, a picture archiving and communication system, or a medical robot. The network unit 130 may transmit various types of data, generated through the operation of the processor 110, through communication with the above-described system, server, or medical robot.

FIG. 2 is a block diagram of a blood vessel detection apparatus according to one embodiment of the present disclosure.

Referring to FIG. 2, a blood vessel detection apparatus 200 includes at least one neural network model, and may receive medical images and detect first blood vessel regions and second blood vessel regions from the medical images. The medical images may be ultrasonic images, but are not limited thereto. The first blood vessels and the second blood vessels may be the same type of blood vessels or different types of blood vessels. More specifically, when the first blood vessels are veins, the second blood vessels may be arteries, and vice versa.

Medical images are input on a per-frame basis for a preset time, and the blood vessel detection apparatus 200 may perform a real-time blood vessel detection operation corresponding to a per-frame basis for the preset time. When the preset time has ended, the blood vessel detection apparatus 200 may perform a final blood vessel detection operation using accumulated medical images.

For example, ultrasonic images may be input on a per-frame basis for a preset time. The blood vessel detection apparatus 200 may detect a first blood vessel region and a second blood vessel region for each frame. These results may be output and displayed on a screen on a per-frame basis. When the preset time has ended, the blood vessel detection apparatus 200 may detect final first blood vessel regions and final second blood vessel regions for the ultrasonic images accumulated during the corresponding time and display them on the screen. In this case, the blood vessel detection apparatus 200 may take into consideration real-time detection results.

The blood vessel detection apparatus 200 may use different neural network models for the real-time blood vessel detection and the final blood vessel detection. Furthermore, the blood vessel detection apparatus 200 may use different neural network models for the first blood vessel detection and the second blood vessel detection.

The medical images acquired during the preset time may vary. For example, when an image acquisition device moves, a contact area and contact pressure for a body part may vary. Accordingly, even when the same body part is imaged, medical images may vary. Accordingly, the blood vessel detection apparatus 200 may perform blood vessel detection on medical images that vary in real time. Furthermore, the blood vessel detection apparatus 200 may perform final blood vessel detection by reflecting the real-time blood vessel detection results therein.

That is, according to the present disclosure, the blood vessel detection apparatus 200 reflects the characteristics of blood vessels varying depending on the imaging environment by performing real-time blood vessel detection, so that the accuracy of blood vessel detection can be increased even in a situation in which it is difficult to acquire consistent images. Furthermore, the blood vessel detection apparatus 200 detects blood vessels by using a plurality of neural network models by taking into consideration different characteristics of blood vessels, so that high-risk blood vessels can be clearly distinguished, thereby preventing the possibility of a medical accident in advance.

Meanwhile, the control conditions of the image acquisition device that generates medical images may be changed by a separate system. The system generates condition change data for the control of the image acquisition device based on the medical images generated by the image acquisition device.

The image acquisition device operates according to a condition control algorithm, and at least one of the hardware characteristics and software characteristics of the image acquisition device is changed according to the condition change data. As an example, the hardware characteristics include at least one of the following: the location of the image acquisition device, the distance from a patient, a body compression intensity for the patient, a compression direction, and a compression time. As an example, the software characteristics include at least one of the following: the time require for acquiring a medical image, and the resolution and size, gain, view depth, spatial compound, and line density of the medical image.

For example, when the image acquisition device is an ultrasound probe, the system may change control conditions such as the distance between the ultrasound probe and a body part, the degree of compression, and the area in contact with the body part. The image acquisition device changes the control conditions and generates ultrasonic images. As a result, the image acquisition device may capture optimal ultrasonic images that enable the detection of blood vessels in the range in which the blood vessels are not compressed.

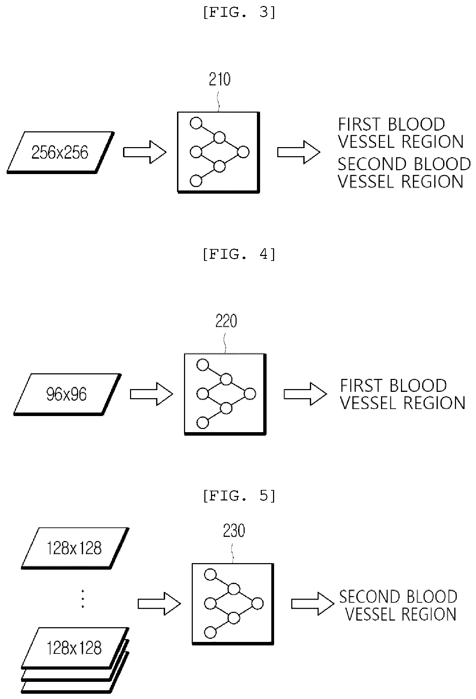

FIG. 3 is a block diagram of a first model according to one embodiment of the present disclosure, FIG. 4 is a block diagram of a second model according to one embodiment of the present disclosure, and FIG. 5 is a block diagram of a third model according to one embodiment of the present disclosure.

Referring to FIG. 3, a first model 210 may detect a blood vessel from a single medical image. That is, the first model 210 may detect a first blood vessel or a second blood vessel. The first model 210 may receive a two-dimensional medical image of a size of 256 × 256 and detect a first blood vessel or second blood vessel included in the medical image. The first model 210 may be used for the real-time blood vessel detection operation of the blood vessel detection apparatus 200.

The second model 220 may detect a first blood vessel from a single medical image. The second model 220 may be used for real-time blood vessel detection operation like the first model 210. Since the second model 220 receives a two-dimensional medical image of a size of 96 × 96, the detection speed may be faster than that of the first model 210. Meanwhile, the second model 220 may also detect a second blood vessel, which may be similar to the first blood vessel detection method.

The blood vessel detection apparatus 200 may maintain the similarity of detection results between frames in real-time blood vessel detection by using the first model 210 and the second model 220 together. The blood vessel detection apparatus 200 may secure the accuracy of blood vessel detection with the first model 210 and secure the detection speed with the second model 220.

The third model 230 may detect a second blood vessel from a plurality of medical images. For example, the third model 230 may receive 96 medical images of a size of 128 × 128. The third model 230 may receive three-dimensional data in which 96 images are stacked and learn the beating characteristics of the second blood vessel.

The corresponding images are accumulated for a preset time, and may be extracted at specific frame intervals from among the medical images input to the blood vessel detection apparatus 200 for the preset time. The third model 230 may be used for the final blood vessel detection operation of the blood vessel detection apparatus 200. Meanwhile, the third model 230 may also detect a first blood vessel, which may be similar to the second blood vessel detection method.

Meanwhile, the sizes of the medical images input to the first model 210 to the third model 230 are not limited thereto.

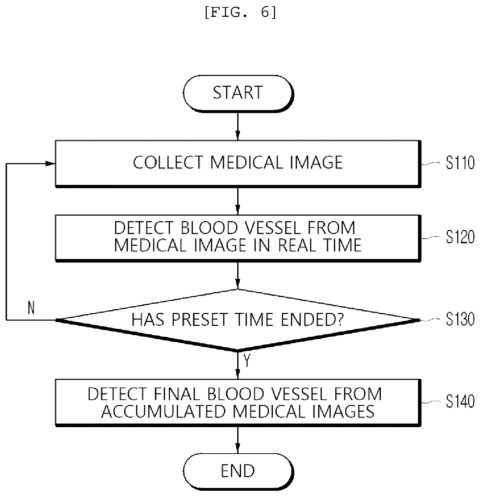

FIG. 6 is a flowchart showing the operation method of a blood vessel detection apparatus according to one embodiment of the present disclosure.

Referring to FIG. 6, the blood vessel detection apparatus 200 may collect a medical image in step S110. As an example, the medical image may be a two-dimensional ultrasonic image, and may be an image acquired by imaging the upper arm area.

The blood vessel detection apparatus 200 may perform real-time blood vessel detection on the medical image in step S120. As an example, the blood vessel detection apparatus 200 may detect a first blood vessel or a second blood vessel from the ultrasonic image.

The blood vessel detection apparatus 200 checks whether a preset time has ended, and returns to step S110 when the preset time has not ended. That is, the blood vessel detection apparatus 200 repeatedly performs real-time blood vessel detection on the medical image for the preset time. Real-time blood vessel detection results may be displayed periodically for the preset time.

When the preset time has ended, the blood vessel detection apparatus 200 may perform final blood vessel detection on accumulated medical images in step S140. As an example, the blood vessel detection apparatus 200 may detect a first blood vessel or a second blood vessel by using at least one medical image from the accumulated medical images.

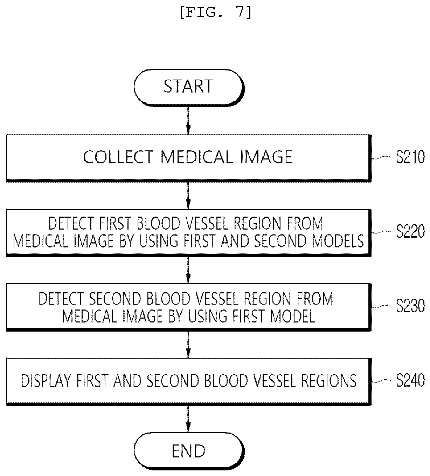

FIG. 7 is a flowchart showing a real-time blood vessel detection method according to one embodiment of the present disclosure.

Referring to FIG. 7, the blood vessel detection apparatus 200 may collect a medical image in step S210.

The blood vessel detection apparatus 200 may detect a first blood vessel region from the medical image by using the first model 210 and the second model 220 in step S220. As an example, the blood vessel detection apparatus 200 may change the size of the medical image and input the resulting medical image to each of the first model 210 and the second model 220. For example, a medical image having a larger size than a medical image input to the second model 220 may be input to the first model 210. Alternatively, a specific portion of a medical image input to the first model 210 may be input to the second model 220. Meanwhile, in step S220, a second blood vessel region may be detected instead of the first blood vessel region by using the first model 210 and the second model 220.

The blood vessel detection apparatus 200 may detect a second blood vessel region from the medical image by using the first model 210 in step S230. Steps S220 and S230 may be performed in parallel. Alternatively, step S230 may be performed before step S220. Meanwhile, in step S230, the first blood vessel region may be detected instead of the second blood vessel region by using the first model 210.

The blood vessel detection apparatus 200 may display the first blood vessel region and the second blood vessel region in step S240. A display method is not limited to a specific one, and the first blood vessel region and the second blood vessel region may be displayed in colors so that they can be distinguished from each other on a medical image.

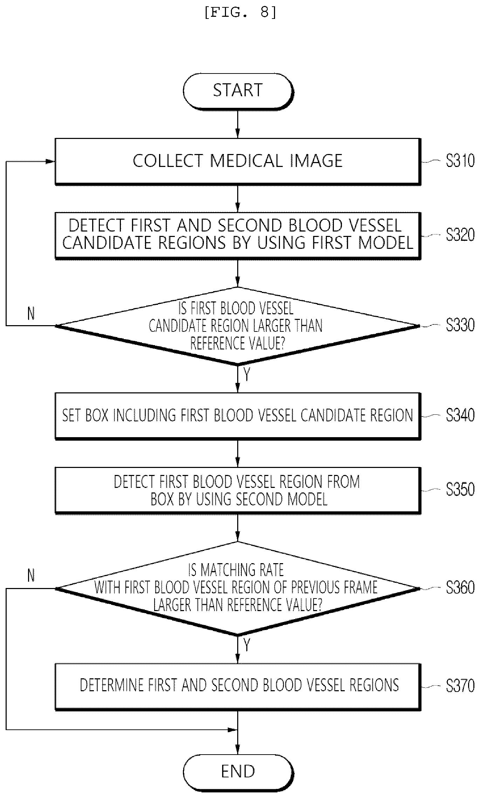

FIG. 8 is a flowchart showing in detail a real-time blood vessel detection method according to one embodiment of the present disclosure.

Referring to FIG. 8, the blood vessel detection apparatus 200 may collect a medical image in step S310. The medical image may constitute one frame.

The blood vessel detection apparatus 200 may detect first and second blood vessel candidate regions from the medical image by using the first model 210 in step S320.

The blood vessel detection apparatus 200 determines whether the first blood vessel candidate region is larger than a reference value in step S330. As an example, the blood vessel detection apparatus 200 determines whether the number of pixels of the first blood vessel candidate region is larger than a reference value. When the number of pixels of the first blood vessel candidate region is not larger than the reference value, the process returns to step S310. The blood vessel detection apparatus 200 performs step S330 so that the blood vessel detection results obtained from consecutive frames are similar to each other between the frames.

Meanwhile, steps S310 to S330 may be repeated a specific number of times, for example, 10 times. In this case, when the number of times that the first blood vessel candidate region is larger than the reference value is larger than a set number of times, step S340 may be entered.

When the first blood vessel candidate region is larger than the reference value, the blood vessel detection apparatus 200 may set a box including the first blood vessel candidate region in step S340. As an example, the blood vessel detection apparatus 200 may set a box including the first blood vessel candidate region and having a size three times that of the first blood vessel candidate region.

The blood vessel detection apparatus 200 may detect a first blood vessel region from the box by using the second model 220 in step S350. As an example, the blood vessel detection apparatus 200 may reset a box depending on the first blood vessel detection results of the second model 220. Meanwhile, the first blood vessel region may include two or more distinct first blood vessels.

The blood vessel detection apparatus 200 determines whether the matching rate with the first blood vessel region of a previous frame is larger than a reference value in step S360. As an example, the blood vessel detection apparatus 200 calculates the matching rate as a score by comparing the first blood vessel region of the frame three frames before a current frame with the first blood vessel region of the current frame. When the score of the matching rate is not larger than the reference value, the blood vessel detection apparatus 200 does not use the first blood vessel region detected from the current frame, but uses the results detected three frames before.

Meanwhile, steps S330 to S360 may also be applied to the second blood vessel. The steps performed to determine the second blood vessel region are similar to the above-described steps, so that detailed descriptions thereof will be omitted. Meanwhile, in step S340, the size of the box set for the second blood vessel may be the same as or different from the box for the first blood vessel. Furthermore, in step S360, the comparison target may be the results obtained three frames before, like in the case of the first blood vessel, or may be a frame other than the frame three frames before a current frame, unlike in the case of the first blood vessel.

In addition, steps S330 to S360 may be performed in parallel for each of the first and second blood vessels. That is, steps S330 to S360 may be performed in parallel according to the type of each blood vessel to determine the first blood vessel region and the second blood vessel region.

When the matching rate is larger than the reference value, the blood vessel detection apparatus 200 may determine the first blood vessel region and the second blood vessel region in the medical image of the current frame in step S370.

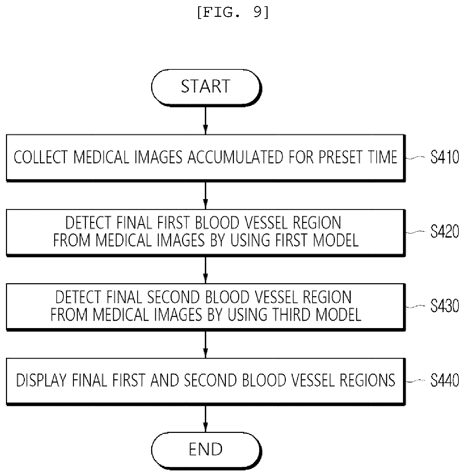

FIG. 9 is a flowchart showing a final blood vessel detection method according to one embodiment of the present disclosure. The steps of FIG. 9 may be performed after step S240 of FIG. 7. For example, the first blood vessel region and the second blood vessel region may be detected for the preset time, and then step S410 may be performed.

Referring to FIG. 9, the blood vessel detection apparatus 200 may collect medical images accumulated for a preset time in step S410.

The blood vessel detection apparatus 200 may detect a final first blood vessel region from the medical images by using the first model 210 in step S420. As an example, the blood vessel detection apparatus 200 may select one of the accumulated medical images and input the selected image to the first model 210. Furthermore, the blood vessel detection apparatus 200 may additionally use the second model 220 to detect a final first blood vessel region. That is, operations similar to steps S340 to S370 may be performed. Meanwhile, in step S420, a second blood vessel region may be detected instead of the first blood vessel region by using the first model.

The blood vessel detection apparatus 200 may detect a final second blood vessel region from the medical images by using the third model 230 in step S430. As an example, the blood vessel detection apparatus 200 may extract medical images from among the accumulated medical images and input them to the third model 230. The frame intervals for the extraction may not be constant. Meanwhile, in step S430, a first blood vessel region may be detected instead of the second blood vessel region by using the third model.

The blood vessel detection apparatus 200 may display the final first blood vessel region and the final second blood vessel region in step S440. A display method is not limited to a specific one, and the final first blood vessel region and the final second blood vessel region may be displayed in colors so that they can be distinguished from each other on a medical image. Meanwhile, when the final first blood vessel region and the final second blood vessel region overlap each other, the blood vessel detection apparatus 200 may display an overlap region as the final second blood vessel region.

The above description of the present disclosure is intended for illustrative purposes, and those having ordinary skill in the art to which the present disclosure pertains will understand that it may be easily modified into other specific forms without changing the technical spirit or essential features of the present disclosure. Therefore, it should be understood that the embodiments described above are exemplary and not limiting in all respects. For example, each component described as a single form may be implemented in a distributed form, and likewise, components described as distributed may be implemented in a combined form.

The scope of the present disclosure is defined by the claims described below rather than the detailed description above, and all changes or modifications derived from the meanings and scope of the claims and their equivalent concepts should be interpreted as being included in the scope of the present disclosure.

## Claims

1. A blood vessel detection method, the blood vessel detection method being performed by a computing device including at least one processor, the blood vessel detection method comprising:
collecting a medical image of an n-th frame (n is a natural number) from an image acquisition device; and
detecting a first blood vessel region from the medical image by using trained first and second models, and detecting a second blood vessel region from the medical image by using the first model;
wherein collecting the medical image and detecting the first and second blood vessel regions are repeated for a preset time.

2. The blood vessel detection method of claim 1, wherein detecting the first and second blood vessel regions comprises:
detecting a first blood vessel candidate region from the medical image by using the first model;
setting a box including the first blood vessel candidate region when the first blood vessel candidate region is larger than a reference value; and
detecting the first blood vessel region from the box by using the second model.

3. The blood vessel detection method of claim 1, further comprising, after detecting the first and second blood vessel regions, displaying the first and second blood vessel regions;
wherein collecting the medical image to displaying the first and second blood vessel regions are repeated for the preset time.

4. The blood vessel detection method of claim 3, wherein displaying the first and second blood vessel regions comprises:
comparing a location of the first blood vessel region with a location of a first blood vessel region of a medical image of a m-th frame (m is a natural number smaller than n), and displaying the first blood vessel region based on comparison results.

5. The blood vessel detection method of claim 4, wherein displaying the first and second blood vessel regions comprises:
comparing a location of the second blood vessel region with a location of a second blood vessel region of the medical image of the m-th frame, and displaying the second blood vessel region based on comparison results.

6. The blood vessel detection method of claim 3, wherein displaying the first and second blood vessel regions comprises:
calculating at least one of a maximum blood vessel cross-sectional area, a degree of blood vessel expansion, and whether a blood vessel is compressed for each of the first and second blood vessel regions; and
displaying at least one of the maximum blood vessel cross-sectional area, the degree of blood vessel expansion, and whether the blood vessel is compressed.

7. The blood vessel detection method of claim 1, further comprising, after the preset time has ended:
detecting a final first blood vessel region from a plurality of medical images accumulated during the preset time by using the first model; and
detecting a final second blood vessel region from the plurality of accumulated medical images by using a trained third model.

8. The blood vessel detection method of claim 7, wherein the third model receives a plurality of medical images extracted from among the plurality of accumulated medical images.

9. The blood vessel detection method of claim 1, wherein the image acquisition device collects the medical image based on a condition control algorithm as at least one of hardware characteristics and software characteristics of the image acquisition device is changed.

10. The blood vessel detection method of claim 9, wherein:
the hardware characteristics include at least one of a location of the image acquisition device, a distance from a patient, a body compression intensity for the patient, a compression direction, and a compression time; and
the software characteristics include at least one of a time taken for acquiring the medical image, and a resolution and size of the medical image.

11. The blood vessel detection method of claim 1,
wherein the first and second blood vessels are a vein and an artery, respectively, or an artery and a vein, respectively.

12. A blood vessel detection method, the blood vessel detection method being performed by a computing device including at least one processor, the blood vessel detection method comprising:
detecting a final first blood vessel region from a plurality of medical images by using a trained first model, and detecting a final second blood vessel region from the plurality of medical images by using a trained second model; and
displaying the final first and second blood vessel regions.

13. The blood vessel detection method of claim 12, further comprising, before detecting the final first and second blood vessel regions, detecting first and second blood vessel regions for a preset time;
wherein the plurality of medical images are acquired for the preset time.

14. The blood vessel detection method of claim 13, wherein detecting the first and second blood vessel regions comprises:
detecting the first blood vessel region from a medical image by using the first model and a trained third model, and detecting the second blood vessel region from the medical image by using the first model.

15. The blood vessel detection method of claim 12, wherein displaying the final first and second blood vessel regions comprises:
displaying a region in which the final first and second blood vessel regions overlap each other as the final second blood vessel region.

16. The blood vessel detection method of claim 12, wherein the trained first model receives any one of the plurality of medical images.

17. The blood vessel detection method of claim 12, wherein the trained second model receives a plurality of medical images extracted from among the plurality of medical images.

18. The blood vessel detection method of claim 12, wherein the plurality of medical images are collected based on a condition control algorithm as at least one of hardware characteristics and software characteristics of an image acquisition device is changed.

19. The blood vessel detection method of claim 12, wherein the first and second blood vessels are a vein and an artery, respectively, or an artery and a vein, respectively.

20. A computer program stored in a computer-readable storage medium, the computer program, when executed on at least one processor, causing the processor to perform operations, wherein the operations comprise:
collecting a medical image from an image acquisition device;
detecting a first blood vessel region from the medical image by using a trained first model and a trained second model, and detecting a second blood vessel region from the medical image by using the first model;
displaying the first blood vessel region and the second blood vessel region;
repeating collecting the medical image and detecting the first and second blood vessel regions for a preset time; and
after the preset time has ended, detecting a final first blood vessel region from a plurality of medical images accumulated during the preset time by using the first model, and detecting a final second blood vessel region from the plurality of accumulated medical images by using the trained third model.
